# FASCICULE DE BREVET EUROPEEN

(11) **EP 0 949 912 B1**
(45) Date de publication et mention de la délivrance du brevet: **18.09.2002**
(21) Numéro de dépôt: 97911313.1
(22) Date de dépôt: 21.10.1997
(51) Int. Cl.: A61K 9/54

(54) **PROCEDE DE PREPARATION D'UNE FORME PHARMACEUTIQUE MULTIPARTICULAIRE A LIBERATION CONTROLEE PLURISEQUENTIELLE**
VERFAHREN ZUR HERSTELLUNG EINER MULTIPARTIKULÄREN PHARMAZEUTISCHEN FORM MITGESTEUERTER ETAPPENWEISER FREISETZUNG
METHOD FOR PREPARING A MULTI-PARTICULATE PHARMACEUTICAL FORM WITH CONTROLLED MULTI-SEQUENTIAL RELEASE

(30) Priorité: 22.10.1996 FR 9612818
(43) Date de publication de la demande: 20.10.1999
(73) Titulaire: ETHYPHARM, 78550 Houdan (FR)
(72) Inventeur: DI COSTANZO, Francis, (Décédé) (FR); COUSIN, Gérard, F-28320 Gallardon (FR); GENDROT, Edouard, F-28500 Vernouillet (FR); CLEE, Marie-Christine, F-28500 Treon (FR)
(74) Mandataire: Koch, Gustave
(86) Numéro de dépôt international: FR9701889
(87) Numéro de publication internationale: WO98017261

(56) Documents cités:
- EP-A- 0 282 698
- EP-A- 0 514 814
- US-A- 4 917 899

## Description

L'invention a pour objet un procédé de préparation d'une forme pharmaceutique multiparticulaire à libération contrôlée pluriséquentielle du genre de celles qui permettent, grâce à seulement une ou deux administrations, d'assurer dans l'organisme du patient pendant 24 heures une concentration suffisante en un ou plusieurs principes actifs.

Les formes pharmaceutiques du genre en question conduisent à l'obtention d'une libération non linéaire *in vitro* du principe actif qui se traduit *in vivo* par la présence d'un ou de plusieurs pics d'absorption du principe actif.

Dans ces formes pharmaceutiques, la substance active est comportée par une pluralité de particules ou sphéroïdes médicamenteux éventuellement à base de centres neutres. Lesdites particules ou sphéroïdes médicamenteux appartiennent respectivement à au moins deux populations ou groupes, chaque population se distinguant par son enrobage dont les caractéristiques physico-chimiques impliquent un profil donné de dissolution in vitro de la matière active; il est possible que l'une des populations ne comporte pas d'enrobage; la granulométrie de ces sphéroïdes médicamenteux est généralement de 0,1 à 1,5 mm.

Les sphéroides médicamenteux appartenant aux différentes populations forment un ensemble remplissant par exemple des gélules ou des comprimés; ils sont distribués de façon homogène au sein de cet ensemble.

Les formes pharmaceutiques du genre en question existent depuis une vingtaine d'années; on connaît en particulier la spécialité commercialisée sous la marque de fabrique SEGLOR qui est à base de dihydroergotamine et qui a été mise sur le marché français dès 1978. Elles peuvent être préparées à l'aide de procédés comportant la mise en oeuvre d'un ensemble d'installations destinées respectivement à la préparation de chacune des populations et au mélange de ces populations une fois préparées.

Ces procédés connus entraînent, de par leur complexité, une augmentation du prix de revient des formes pharmaceutiques en question en raison du nombre d'opérations et d'installations mises en oeuvre.

La préoccupation permanente de l'industrie pharmaceutique étant d'optimiser le prix de revient des médicaments, la Société Demanderesse s'est donné comme but de mettre au point un procédé simplifié propre à conduire à des formes pharmaceutiques du genre en question dont les qualités intrinsèques sont au moins équivalentes à celles des formes pharmaceutiques qui existent déjà mais qui sont obtenues à un coût unitaire sensiblement inférieur.

Et elle a eu le mérite, au terme de recherches approfondies, de mettre au point un procédé répondant à ce but.

Ce procédé est caractérisé par le fait que la forme pharmaceutique du genre en question, qui comporte au moins deux populations de particules distinctes par leur enrobage, est préparée, de préférence en continu, à l'intérieur d'une seule et unique installation d'enrobage à partir d'une pluralité de sphéroïdes médicamenteux semblables et d'un ou plusieurs produits d'enrobage, la pluralité de sphéroïdes médicamenteux étant divisée en autant de fractions que de populations devant être comportées par la forme pharmaceutique à préparer, les fractions de sphéroïdes médicamenteux ainsi obtenues étant introduites successivement dans l'unique installation d'enrobage pour y être soumises, sauf éventuellement la dernière, à un traitement d'enrobage à l'aide d'autant de fractions de l'un ou de l'autre des susdits produits d'enrobage, l'enrobage d'une fraction donnée de sphéroïdes médicamenteux étant effectué en présence des sphéroïdes médicamenteux de la ou des fraction(s) précédente(s) qui ont déjà été soumis à un traitement d'enrobage, la pluralité de sphéroïdes médicamenteux ainsi traités constituant un ensemble au sein duquel il y a une répartition homogène des sphéroides médicamenteux de chaque fraction ou population, les sphéroïdes de chaque fraction, sauf éventuellement l'une d'entre elles, comportant un enrobage qui leur est propre, ladite pluralité de particules étant ensuite mise en forme de façon à constituer la forme pharmaceutique recherchée.

Pour préparer les sphéroïdes médicamenteux, on peut faire comporter une substance active à des centres neutres; à cette fin, on peut avoir recours à l'un ou l'autre des procédés connus suivants:
- application de la substance active sur centre neutre en turbine
- application de la substance active sur centre neutre en lit fluidisé.

Il est également possible de préparer les sphéroides médicamenteux sans utilisation de centres neutres, soit par rotogranulation directe de la substance active en lit d'air fluidisé, soit par un procédé d'extrusion-sphéronisation.

L'installation unique d'enrobage des sphéroïdes médicamenteux mise en oeuvre dans le procédé conforme à l'invention fonctionne en continu ou en discontinu suivant le principe du lit fluidisé ou selon le principe de la turbine classique.

Dans le cas des installations fonctionnant suivant le principe du lit fluidisé, celles-ci peuvent être constituées par un dispositif du type de ceux connus dans le métier par l'appellation "fluid bed coater", par exemple celui commercialisé par la Société Glatt sous l'appellation GPCG, celui commercialisé par la Société Hüttlin sous l'appellation "Kugel-Coater" ou celui commercialisé par la Société Freund sous l'appellation "Flow coater".

Dans le cas des installations fonctionnant suivant le principe de la turbine classique, on peut avoir recours aux turbines commercialisées par la Société Mastra.

L'invention pourra être encore mieux comprise à l'aide du complément de description qui suit et des exemples non limitatifs et relatifs à des modes de réalisation avantageux.

On rappelle que le principe général de la libération des principes actifs à partir d'un sphéroide médicamenteux enrobé ou non répond à deux grandes catégories de phénomènes:
- l'érosion du polymère d'enrobage avec libération subséquente du principe actif,
- la diffusion du principe actif au travers d'une membrane d'enrobage et/ou d'une matrice réticulée.

Les mélanges de sphéroides médicamenteux comportant au moins deux populations ou fractions de ces sphéroides dont au moins l'une est à libération rapide et dont au moins une autre est à libération lente, sont utilisés de façon classique pour permettre l'administration simultanée grâce à une seule et même forme pharmaceutique d'une dose dite "dose d'attaque" de principe actif (fraction rapide) et d'une dose dite "dose d'entretien" de principe actif (fraction lente).

Les formes pharmaceutiques de ce genre permettent la réalisation de protocoles thérapeutiques tels que, dans l'organisme du patient, une concentration minimale thérapeutique en un ou plusieurs principes actifs est maintenue en permanence en ayant recours à seulement deux, voire une administration(s) par jour.

Se proposant de préparer une telle forme pharmaceutique, conformément à l'invention on s'y prend comme suit ou de façon équivalente.

On prépare d'abord des sphéroides médicamenteux non enrobés.

Pour ce faire, on peut procéder, comme indiqué dans le brevet U.S. 5.229.135 au nom de la Société Demanderesse, en fixant un principe actif donné, qui est généralement sous la forme d'une poudre de granulométrie inférieure à 100 µm, sur des centres neutres, de préférence sphériques, d'une granulométrie généralement d'environ 0,1 à environ 1,5 mm et, de préférence, de 0,4 à 0,7 mm et constitués avantageusement de saccharose et d'amidon.

De tels centres neutres se trouvent dans le commerce sous l'appellation "non-pareils" et sont fabriqués et commercialisés entre autres par la Société Mendell.

Les centres neutres peuvent être également constitués avantageusement par des cristaux du principe actif lui-même ou par des excipients à usage pharmaceutique tels que le saccharose, le lactose ou encore la cellulose; la granulométrie de ces centres neutres est généralement d'environ 0,05 à environ 0,30 mm et, de préférence, de 0,15 à 0,20 mm.

Pour réaliser la fixation du principe actif sur les centres neutres, on peut déposer sur ceux-ci tout d'abord une pellicule d'une solution liante puis une couche de substance active pulvérulente, les centres neutres ainsi traités étant soumis à une étape de séchage subséquente; il est également possible de fixer le principe actif par application d'une suspension de ce principe actif dans la solution liante. La composition de la suspension relève des connaissanges générales de l'homme du métier.

Cette séquence d'opérations est répétée autant de fois qu'il faut pour obtenir la fixation sur chaque centre neutre de la quantité souhaitée de substance active.

Pour terminer la préparation, on recouvre les sphéroides médicamenteux obtenus d'une pellicule polymérique et on les saupoudre avec un agent antimottant, par exemple du talc.

On dispose ainsi d'une population homogène de sphéroïdes médicamenteux comportant une substance active donnée.

C'est à cette population homogène de sphéroïdes médicamenteux qu'on applique le procédé conforme à l'invention.

Dans ce but, on divise la susdite population homogène de sphéroïdes médicamenteux, qui constitue un lot, en au moins deux fractions dont les importances respectives sont déterminées en fonction du profil de dissolution in vitro que l'on désire obtenir.

De façon préférentielle, on prévoit deux fractions.

On sélectionne par ailleurs une ou plusieurs compositions d'enrobage qui sont généralement à base d'une ou plusieurs résines acryliques et/ou méthacryliques en solution dans des solvants du groupe comprenant préférentiellement l'éthanol, l'acétone, l'isopropanol et l'eau à l'état pur ou en mélange ; ces compositions peuvent comprendre des agents plastifiants, lubrifiants et anti-adhérents.

L'enrobage est effectué, conformément à l'invention, à l'aide d'une seule et unique installation comportant, d'une part, des moyens propres à mettre les sphéroides médicamenteux soit sous forme de lit fluidisé, soit en rotation comme dans une turbine classique et, d'autre part, des moyens de pulvérisation des compositions d'enrobage.

L'installation en question peut être constituée par le dispositif connu sous l'expression de métier "fluid bed coater" (dispositif à enrobage en lit fluidisé), par exemple celui commercialisé par la Société GLATT sous la désignation GPCG.

Dans un premier temps, on introduit dans cette installation unique l'une des susdites fractions de sphéroides médicamenteux; ces derniers sont mis sous forme de lit fluidisé et une première quantité d'une première composition d'enrobage est appliquée par pulvérisation sur les sphéroïdes en lit fluidisé.

Dans un deuxième temps, on introduit dans l'installation, à l'intérieur de laquelle sont maintenus en lit fluidisé les sphéroïdes médicamenteux de la première fraction qui viennent d'être pourvus d'un enrobage, les sphéroïdes médicamenteux d'une deuxième fraction du susdit lot.

Les sphéroïdes de la deuxième fraction sont mis sous forme de lit fluidisé en présence des sphéroïdes déjà enrobés de la première fraction, le lit fluidisé résultant étant composé de sphéroides revêtus et de sphéroïdes non encore revêtus.

On applique alors sur le lit fluidisé ainsi constitué une deuxième quantité de la susdite première composition d'enrobage ou une première quantité d'une deuxième composition d'enrobage.

Cette nouvelle quantité de composition d'enrobage est appliquée toujours par pulvérisation sur le lit fluidisé de sphéroides déjà enrobés et de sphéroides sans enrobage.

On obtient ainsi un lot de sphéroïdes médicamenteux comportant une première population de sphéroïdes revêtus des deux enrobages successifs appliqués lors des première et deuxième opérations d'enrobage et une deuxième population de sphéroïdes revêtus d'un seul enrobage appliqué lors de la deuxième opération d'enrobage.

Dans l'éventualité où, au départ, on a divisé la population initiale de sphéroïdes médicamenteux non enrobés en trois fractions, on introduit alors la troisième fraction dans l'installation à l'intérieur de laquelle sont maintenues sous forme de lit fluidisé les susdites première et seconde populations qui se distinguent l'une de l'autre par leurs enrobages successifs.

Les sphéroïdes de cette troisième fraction non encore enrobés sont donc mis sous forme de lit fluidisé en présence des sphéroïdes déjà enrobés des susdites première et deuxième populations.

L'ensemble ainsi constitué peut soit être utilisé tel quel pour remplir par exemple des gélules ou des comprimés, soit être soumis à une troisième opération d'enrobage à l'aide d'une troisième fraction de la première composition d'enrobage ou d'une deuxième fraction de la deuxième composition d'enrobage, ou encore d'une première fraction d'une troisième composition d'enrobage.

Quelle que soit la solution adoptée, on dispose alors d'un ensemble homogène de sphéroïdes médicamenteux composé de populations différant les unes des autres par leurs enrobages successifs et douées par conséquent de vitesses propres à chacune d'elles de libération *in vitro* du principe actif, conduisant au profil de dissolution homogène recherché.

La cinétique de la libération de la substance active de l'ensemble ainsi constitué dépend
- de l'importance respective des différentes fractions en lesquelles a été divisé le lot initial de sphéroïdes médicamenteux non enrobés et
- de la nature et des quantités de composition d'enrobage mises en oeuvre à chaque opération dans la mesure où ces quantités proviennent d'une seule et même composition d'enrobage ou de la nature de chaque composition d'enrobage et de la quantité mise en oeuvre de chaque composition dans la mesure où plusieurs compositions d'enrobage différentes les unes des autres sont utilisées.

La détermination de ces paramètres, en vue de l'obtention d'une cinétique de libération *in vitro* donnée de la substance active, relève des connaissances générales de l'homme du métier.

La masse totale de résine entrant dans la constitution de la composition d'enrobage, en d'autres termes la masse totale de mélange polymérique devant être appliquée à l'ensemble du lot de sphéroïdes médicamenteux, est définie en fonction de la cinétique de libération du principe actif souhaitée; la masse de mélange polymérique apportée par chaque fraction de composition d'enrobage est définie de telle sorte que la somme des masses apportées par les différentes fractions soit égale à la masse totale dont il a été question ci-dessus.

Les exemples qui suivent illustrent la détermination des paramètres en question.

Le fonctionnement de l'installation unique peut être en continu, les charges successives de sphéroïdes médicamenteux non enrobés en alternance avec les opérations d'enrobage étant introduites successivement, par exemple sous dépression.

Il peut également être en discontinu, l'introduction des sphéroïdes non enrobés et des compositions d'enrobage étant réalisée par charges.

### EXEMPLE 1

Préparation de sphéroïdes médicamenteux enrobés, constitutifs de la forme pharmaceutique selon l'invention et dont le principe actif est constitué par le chlorhydrate de Diltiazem ou (2S-cis)-3-(acétyloxy)-5-[2-(diméthylamino) éthyl]-2,3-dihydro-2-(4-méthoxyphényl)-1,5-benzothiazépine-4-(5H)-one.

Dans une première étape, on prépare les noyaux contenant le principe actif, autrement dit les sphéroïdes médicamenteux.

Le principe actif est fixé sur des supports ou centres neutres sphériques constitués de saccharose et d'amidon; leur diamètre moyen est compris entre 0,4 et 0,7 mm.

On utilise une turbine conventionnelle et une solution liante contenant, dans de l'éthanol, 20% en poids sec d'un mélange de polymères comprenant, d'une part, une résine acrylique, par exemple celle connue sous la marque de fabrique EUDRAGIT RS et, d'autre part, de la polyvidone, les deux polymères étant en des proportions pondérales respectives de 75/25.

Pour fixer la substance active sur les centres neutres, on pulvérise un volume mesuré de la susdite solution liante sur lesdits centres, puis on apporte par poudrage une quantité définie de principe actif.

Le cycle comprenant la pulvérisation de la solution liante et le poudrage du principe actif ainsi qu'une étape de séchage, est répété autant de fois qu'il est nécessaire pour fixer la totalité préalablement définie du principe actif sur les centres neutres sphériques.

Les sphéroïdes médicamenteux ainsi préparés sont mis à sécher à la température ambiante dans la même turbine pendant environ 12 heures.

On applique ensuite sur les sphéroïdes séchés, précédemment obtenus, une pellicule polymérique par pulvérisation d'une solution alcoolique de la susdite résine acrylique pouvant être constituée par celle connue sous la marque EUDRAGIT RS (15 à 20% en poids sec); puis on applique par saupoudrage une quantité définie de talc et on sèche.

Ce cycle "pulvérisation-poudrage-séchage" est répété 12 fois dans la même turbine.

Les sphéroïdes ainsi obtenus, qui forment un lot d'homogénéité acceptable du point de vue de la teneur en principe actif, sont mis en oeuvre dans le procédé conforme à l'invention.

C'est donc dans une deuxième étape qu'est mis en oeuvre ledit procédé en vue de l'enrobage des sphéroïdes médicamenteux contenant le principe actif.

Le lot homogène de sphéroïdes médicamenteux contenant le principe actif, c'est-à-dire du Diltiazem HCl, est divisé en deux fractions dont les importances respectives sont déterminées au préalable en fonction du profil final de dissolution *in vitro* recherché.

Dans le cas de l'exemple, les deux fractions représentent respectivement 60 et 40% du lot.

La première fraction qui contient 60% de la masse des sphéroïdes médicamenteux, est introduite dans la cuve d'une installation d'enrobage à lit fluidisé, équipée d'une colonne Würster.

De préférence, la valeur absolue de la masse de cette première fraction du susdit lot ne représente pas plus de 40% de la capacité de traitement de l'installation; elle doit par ailleurs être suffisante pour permettre la constitution d'un lit fluidisé.

Sur cette première fraction de noyaux actifs mis en lit fluidisé, on applique par pulvérisation une première partie, c'est-à-dire 44% en masse de la quantité totale d'une composition d'enrobage constituée par un mélange de polymères et d'agents plastifiants, anti-adhérents et lubrifiants.

Cette première partie de la composition d'enrobage est appliquée sous la forme d'une suspension à 15% de matière sèche au sein d'un mélange de solvants "éthanol/acétone (60/40; m/m)"; le mélange de polymères est constitué de résines acryliques, à savoir celles connues sous les marques EUDRAGIT RS et EUDRAGIT RL, en des proportions pondérales de 95/5; les agents plastifiants, anti-adhérents et lubrifiants sont respectivement constitués par du phtalate d'éthyle, de la silice colloïdale de marque AEROSIL R 972 et du talc.

On pulvérise la totalité de cette suspension sur les sphéroïdes de la première fraction.

On introduit ensuite la deuxième fraction de sphéroïdes médicamenteux (40% du lot) dans la cuve de l'installation qui contient déjà, sous forme de lit fluidisé, les sphéroïdes médicamenteux enrobés de la première fraction.

Le lit fluidisé résultant se compose donc de sphéroïdes médicamenteux déjà pourvus d'un enrobage (60% du total) et de sphéroïdes non encore enrobés (40% du total), les sphéroïdes enrobés et ceux qui ne le sont pas encore étant distribués de manière homogène au sein du lit fluidisé.

Sur ce lit fluidisé ainsi constitué, on applique par pulvérisation la deuxième partie, c'est-à-dire les 56% en poids restants de la susdite composition à base de polymères devant être appliquée sur l'ensemble du lot de sphéroïdes.

Cette deuxième partie de la composition d'enrobage est appliquée sous la forme d'une seconde suspension à 15% de matière sèche au sein d'un mélange de solvants "éthanol/acétone (60/40; m/m)"; cette fois-ci, le mélange de polymères comprend les deux résines acryliques susmentionnées EUDRAGIT RS et EUDRAGIT RL en des proportions de 90/10.

Après pulvérisation de la totalité de cette deuxième partie de la composition d'enrobage, le lot de sphéroïdes médicamenteux est mis à sécher dans l'installation pendant environ 30 minutes à une température de 40°C.

Une fois le séchage terminé, on prélève un certain nombre de sphéroïdes du lot et on détermine leur courbe de dissolution.

Pour ce faire, on utilise un appareil de dissolution à palette correspondant à celui désigné sous l'appellation Appareil I de la Pharmacopée US n° XXIII; on utilise de l'eau purifiée comme milieu de dissolution; les conditions d'utilisation de l'appareil sont de 900 ml d'eau purifiée et 50 tours/minute.

On détermine sur quatre échantillons toutes les heures de la 1ère à la 12ème et toutes les deux heures de la 12ème à la 24ème:
- la quantité de principe actif libéré (exprimée en % en poids) par rapport à la quantité totale de principe actif comporté par un échantillon donné et
- la déviation standard (Sd) des mesures.

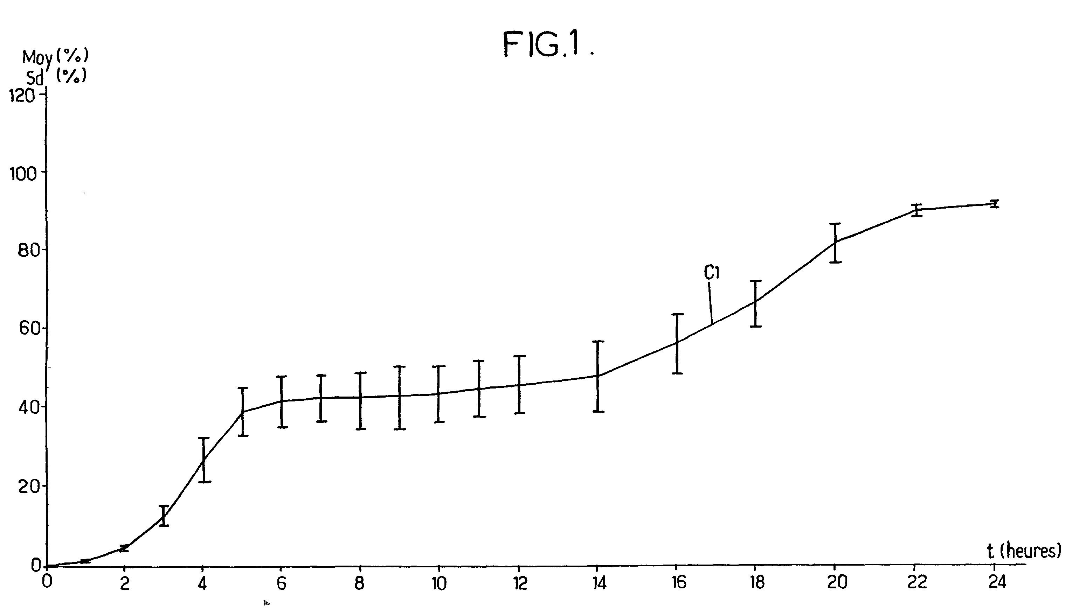

Les moyennes (Moy) des résultats obtenus sur les quatre échantillons et les valeurs de Sd correspondantes sont réunies dans le tableau I ci-après et visualisées par la courbe C₁ montrée à la figure 1. Cette courbe C₁ représente la variation de principe actif libéré en fonction du temps t (exprimé en heures).

De l'examen de cette courbe C₁, il résulte que la cinétique de dissolution du principe actif présente deux séquences successives s'étalant sur 24 heures.

**TABLEAU I**

| | | | | | | | | | |
|---|---|---|---|---|---|---|---|---|---|
| t (heures) | 1:00 | 2:00 | 3:00 | 4:00 | 5:00 | 6:00 | 7:00 | 8:00 | 9:00 |
| Moy (%) | 1,27 | 4,19 | 12,33 | 26,18 | 38,67 | 41,27 | 42,11 | 42,10 | 42,32 |
| Sd (%) | 0,21 | 0,73 | 2,03 | 4,30 | 4,41 | 4,78 | 4,59 | 5,10 | 5,45 |
| t (heures) | 10:00 | 11:00 | 12:00 | 14:00 | 16:00 | 18:00 | 20:00 | 22:00 | 24:00 |
| Moy (%) | 42,73 | 44,03 | 44,36 | 47,19 | 55,38 | 66,03 | 81,29 | 89,48 | 91,13 |
| Sd (%) | 5,03 | 5,11 | 5,20 | 6,56 | 5,53 | 4,15 | 3,55 | 0,97 | 0,91 |

### EXEMPLE 2

Préparation de sphéroïdes médicamenteux enrobés, constitutifs de la forme pharmaceutique selon l'invention et dont le principe actif est constitué par le chlorhydrate de Verapamil ou α[3-[[2-(3,4-diméthoxyphényl)éthyl]méthylamino]propyl]-3,4-diméthoxy-α-(1-méthyléthyl)-benzèneacétonitrile.

Dans une première étape, on prépare les noyaux contenant le principe actif, autrement dit les sphéroïdes médicamenteux.

Le principe actif est fixé sur des supports ou centres neutres sphériques constitués de saccharose et d'amidon de diamètre moyen compris entre 0,4 et 0,7 mm, en ayant recours à l'installation devant servir à l'enrobage subséquent, c'est-à-dire à une installation d'enrobage à lit fluidisé équipée d'une colonne Würster; le principe actif est amené sur un lit fluidisé de centres neutres, établi à l'intérieur de l'installation, par pulvérisation d'une suspension liante contenant 34% en poids sec d'un mélange constitué de Vérapamil HCl, de polyvidone et de polyéthylèneglycol 400 dans un mélange solvant d'isopropanol et d'éthanol en proportions égales.

A la fin de la pulvérisation de la suspension, les microgranules sont séchés dans l'installation pendant 30 minutes à la température de 40°C.

Dans une deuxième étape, les sphéroïdes médicamenteux ainsi obtenus, qui forment un lot d'homogénéité acceptable du point de vue de la quantité de principe actif, sont mis en oeuvre dans le cadre du procédé conforme à l'invention.

Pour ce faire, le lot homogène de sphéroïdes médicamenteux contenant du Vérapamil HCl est retiré de l'installation et divisé en deux fractions dont les importances respectives sont déterminées au préalable en fonction du profil final de dissolution in *vitro* recherché.

Dans le cas de l'exemple, les deux fractions représentent respectivement 60 et 40% du lot.

La première fraction qui contient 60% de la masse des sphéroïdes médicamenteux, est réintroduite dans la cuve de la même installation d'enrobage à lit fluidisé, équipée d'une colonne Würster.

De préférence, la valeur absolue de la masse de cette première fraction du susdit lot ne représente pas plus de 40% de la capacité de traitement de l'installation; elle doit par ailleurs être suffisante pour permettre la constitution d'un lit fluidisé.

Sur cette première fraction de sphéroïdes médicamenteux mis en lit fluidisé, on applique par pulvérisation une première partie, c'est-à-dire 42% en poids de la quantité totale d'une composition d'enrobage constituée par un mélange de polymères et d'agents plastifiants, anti-adhérents et lubrifiants.

Cette première partie de la composition d'enrobage est appliquée sous la forme d'une suspension à 15% de matière sèche au sein d'un mélange de solvants "éthanol/acétone (60/40; m/m)"; le mélange de polymères est constitué de résines acryliques, à savoir celles connues sous les marques EUDRAGIT RS et EUDRAGIT RL, en des proportions pondérales de 90/10; les agents plastifiants, anti-adhérents et lubrifiants sont respectivement constitués par du phtalate d'éthyle, de la silice de marque AEROSIL R 972 et du talc.

On pulvérise la totalité de cette suspension sur les sphéroïdes de la première fraction.

On introduit ensuite la deuxième fraction de sphéroïdes médicamenteux (40% du lot) dans la cuve de l'installation qui contient déjà, sous forme de lit fluidisé, les sphéroides médicamenteux enrobés de la première fraction de la composition d'enrobage.

Le lit fluidisé résultant se compose donc de sphéroïdes médicamenteux déjà pourvus d'un enrobage (60% du total) et de sphéroïdes non encore enrobés (40% du total), les sphéroïdes enrobés et ceux qui ne le sont pas encore étant distribués de manière homogène au sein du lit fluidisé.

Sur ce lit fluidisé ainsi constitué, on applique par pulvérisation la deuxième partie, c'est-à-dire les 58% en poids restants de la susdite composition à base de polymères devant être appliquée sur l'ensemble du lot de sphéroïdes.

Cette deuxième partie de la composition d'enrobage est appliquée sous la forme d'une seconde suspension à 15% de matière sèche au sein d'un mélange de solvants "éthanol/acétone (60/40; m/m)"; cette fois-ci, le mélange de polymères comprend encore les deux résines acryliques susmentionnées EUDRAGIT RS et EUDRAGIT RL en des proportions de 90/10.

Après pulvérisation de la totalité de cette deuxième partie de la composition d'enrobage, le lot de sphéroïdes médicamenteux est mis à sécher dans l'installation pendant environ 30 minutes à une température de 40°C.

Une fois le séchage terminé, on prélève un certain nombre de sphéroides du lot et on détermine leur courbe de dissolution.

Pour ce faire, on utilise un appareil de dissolution à palette correspondant à celui désigné sous l'appellation Appareil I de la Pharmacopée US n° XXIII; on utilise de l'eau purifiée comme milieu de dissolution; les conditions d'utilisation de l'appareil sont de 900 ml d'eau purifiée et de 100 tours/minute.

On détermine sur trois échantillons toutes les heures de la 1ère à la 12ème:
- la quantité de principe actif libéré (exprimée en % en poids) par rapport à la quantité totale de principe actif comporté par un échantillon donné et
- la déviation standard (Sd) des mesures.

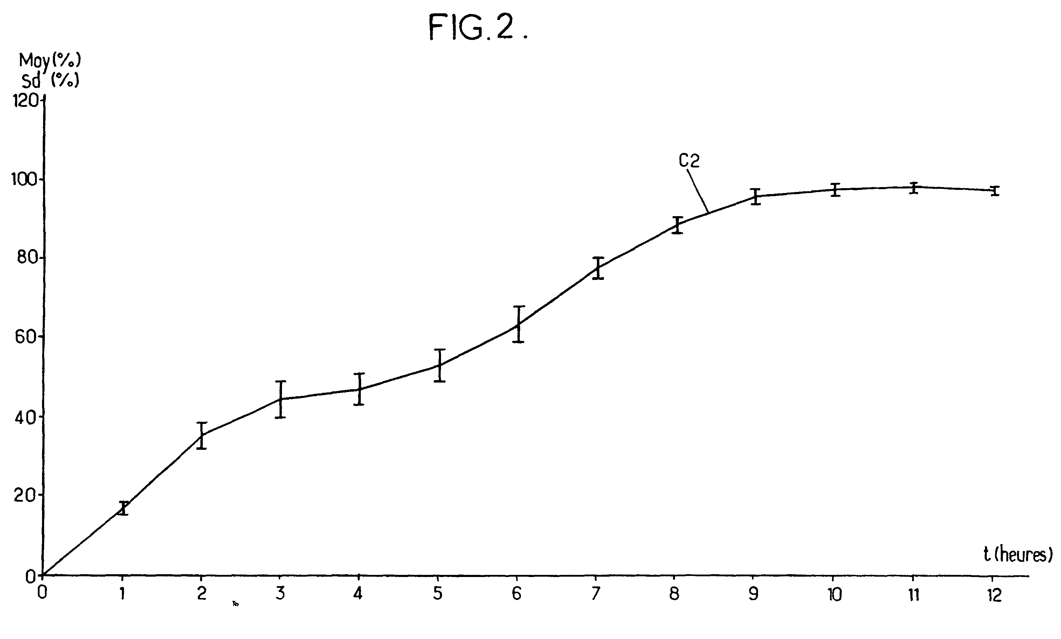

Les moyennes (Moy) des résultats obtenus sur les trois échantillons et les valeurs de Sd correspondantes sont réunies dans le tableau II ci-après et visualisées par la courbe C₂ montrée à la figure 2. Cette courbe C₂ représente la variation de principe actif libéré (%) en fonction du temps t (exprimé en heures).

De l'examen de cette courbe C₂, il résulte que la cinétique de dissolution du principe actif présente deux séquences successives s'étalant sur 12 heures.

**TABLEAU II**

| | | | | | | |
|---|---|---|---|---|---|---|
| t (heures) | 1:00 | 2:00 | 3:00 | 4:00 | 5:00 | 6:00 |
| Moy (%) | 16,87 | 35,33 | 43,77 | 46,27 | 51,33 | 62,40 |
| Sd (%) | 1,32 | 3,31 | 4,51 | 3,70 | 4,10 | 4,84 |
| t (heures) | 7:00 | 8:00 | 9:00 | 10:00 | 11:00 | 12:00 |
| Moy (%) | 77,27 | 88,30 | 95,57 | 97,03 | 97,70 | 96,97 |
| Sd (%) | 2,58 | 1,91 | 1,89 | 1,33 | 1,00 | 1,06 |

## Revendications

1. Procédé de préparation d'une forme pharmaceutique multiparticulaire à libération contrôlée pluriséquentielle permettant d'assurer dans l'organisme du patient une concentration suffisante en un ou plusieurs principes actifs par une ou deux administrations en 24 heures, **caractérisé par le fait que** la forme pharmaceutique du genre en question, qui comporte au moins deux populations de particules distinctes par leur enrobage, est préparée, de préférence en continu, à l'intérieur d'une seule et unique installation d'enrobage à partir d'une pluralité de sphéroïdes médicamenteux semblables et d'un ou plusieurs produits d'enrobage, la pluralité de sphéroïdes médicamenteux étant divisée en autant de fractions que de populations devant être comportées par la forme pharmaceutique à préparer, les fractions de sphéroïdes médicamenteux ainsi obtenues étant introduites successivement dans l'unique installation d'enrobage pour y être soumises, sauf éventuellement la dernière, à un traitement d'enrobage à l'aide d'autant de fractions de l'un ou de l'autre des susdits produits d'enrobage, l'enrobage d'une fraction donnée de sphéroïdes médicamenteux étant effectué en présence des sphéroïdes médicamenteux de la ou des fraction(s) précédente(s) qui ont déjà été soumis à un traitement d'enrobage, la pluralité de sphéroïdes médicamenteux ainsi traités constituant un ensemble au sein duquel il y a une répartition homogène des sphéroïdes médicamenteux de chaque fraction ou population, les sphéroïdes de chaque fraction, sauf éventuellement l'une d'entre elles, comportant un enrobage qui leur est propre, ladite pluralité de particules étant ensuite mise en forme de façon à constituer la forme pharmaceutique recherchée.

2. Procédé selon la revendication 1, **caractérisé par le fait que** l'installation unique d'enrobage des sphéroïdes médicamenteux fonctionne en continu ou en discontinu suivant le principe du lit fluidisé et est constituée par un dispositif du type de ceux connus dans le métier sous l'appellation "fluid bed coater".

3. Procédé selon la revendication 1, **caractérisé par le fait que** l'installation unique d'enrobage des sphéroïdes médicamenteux fonctionne en continu ou en discontinu suivant le principe de la turbine classique.

4. Procédé selon la revendication 1, **caractérisé par le fait qu'**on réalise la fabrication des sphéroïdes médicamenteux à partir des centres neutres dans l'installation unique par application en lit fluidisé de la substance active.

## Patentansprüche

1. Verfahren zur Herstellung einer multipartikulären pharmazeutischen Form mit gesteuerter etappenweiser Freisetzung, welche ermöglicht mittels einer oder zwei Verabreichungen innerhalb 24 Stunden eine ausreichende Konzentration eines oder mehrerer Wirkstoffe im Organismus eines Patienten zu gewährleisten, **dadurch gekennzeichnet, daß** die pharmazeutische Form der fraglichen Art, welche mindestens zwei hinsichtlich ihrer Umhüllung verschiedene Partikelpopulationen umfaßt, bevorzugt kontinuierlich im Inneren einer einzigen Umhüllungseinrichtung hergestellt wird, ausgehend von mehreren ähnlichen Arzneimittelkügelchen und einer oder mehreren Umhüllungssubstanzen, wobei die mehreren Arzneimittelkügelchen in so viele Fraktionen aufgeteilt werden wie die herzustellende pharmazeutische Form umfassen soll, die derart erhaltenen Arzneimittelkügelchenfraktionen nacheinander in die einzige Umhüllungseinrichtung eingebracht werden, worin sie, gegebenenfalls mit Ausnahme der letzten Fraktion, einer Umhüllungsbehandlung mit ebenso vielen Fraktionen einer oder mehrerer der Umhüllungssubstanzen unterzogen werden, wobei das Umhüllen einer gegebenen Arzneimittelkügelchenfraktion in der Gegenwart der vorhergehenden Fraktion oder von vorhergehenden Fraktionen, welche bereits einer Umhüllungsbehandlung unterzogen wurde bzw. wurden, ausgeführt wird, die derart behandelten mehreren Arzneimittelkügelchen eine Gesamtheit mit einer homogenen Verteilung an Arzneimittelkügelchen jeder Fraktion oder Population bilden, die Kügelchen jeder Fraktion, gegebenenfalls mit einer einzigen Ausnahme, eine ihnen eigene Umhüllung aufweisen, und wobei die mehreren Teilchen danach in Form gebracht werden um die gewünschte pharmazeutische Form zu bilden.

2. Verfahren nach Anspruch 1, **dadurch gekennzeichnet, daß** die einzige Einrichtung zum Umhüllen der Arzneimittelkügelchen kontinuierlich oder diskontinuierlich nach dem Fließbettprinzip arbeitet und aus einer Vorrichtung des Typs gebildet ist, der in der Technik unter der Bezeichnung "fluid bed coater" bekannt ist.

3. Verfahren nach Anspruch 1, **dadurch gekennzeichnet, daß** die einzige Einrichtung zum Umhüllen der Arzneimittelkügelchen kontinuierlich oder diskontinuierlich nach dem Prinzip der klassischen Turbine arbeitet.

4. Verfahren nach Anspruch 1, **dadurch gekennzeichnet, daß** man die Herstellung von Arzneimittelkügelchen in der einzigen Einrichtung ausgehend von neutralen Kernen mittels Aufbringen der Wirksubstanz im Fließbett bewirkt.

## Claims

1. A process for the production of a multisequential controlled-release, multiparticulate dosage form which makes it possible to provide a sufficient concentration of one or more active ingredients in the patient's body by administration of one or two doses in 24 hours, **characterised in that** the dosage form of the type in question, which comprises at least two populations of particles distinguished by the coating thereof, is produced, preferably continuously, within one single coating installation from a plurality of similar medicinal spheroids and one or more coating products, the plurality of medicinal spheroids being divided into as many fractions as the dosage form to be produced is to comprise populations, the fractions of medicinal spheroids so obtained being introduced in succession into the single coating installation in order to be subjected therein, with the possible exception of the last fraction, to a coating treatment by means of as many fractions of one or the other of said coating products, coating of a given fraction of medicinal spheroids being carried out in the presence of the medicinal spheroids of the preceding fraction(s) which have already been subjected to coating treatment, the plurality of medicinal spheroids treated in said manner constituting a set within which there is a uniform distribution of the medicinal spheroids from each fraction or population, the spheroids of each fraction, with the possible exception of one fraction, comprising a coating appropriate therefor, said plurality of particles then being shaped in such a manner as to constitute the desired pharmaceutical form.

2. A process according to claim 1, **characterised in that** the single coating installation for the medicinal spheroids operates continuously or batchwise in accordance with the fluidised bed principle and consists of an apparatus of the type known in the art as a "fluid bed coater".

3. A process according to claim 1, **characterised in that** the single coating installation for the medicinal spheroids operates continuously or batchwise in accordance with the conventional turbine principle.

4. A process according to claim 1, **characterised in that** the medicinal spheroids are produced from neutral cores in the single installation by fluidised bed application of the active substance.
